# EUROPEAN PATENT APPLICATION

(11) **EP 1 712 614 A1**
(43) Date of publication of application: **18.10.2006**
(21) Application number: 05709916.0
(22) Date of filing: 02.02.2005
(51) Int. Cl.: C12M 1/34, C12M 1/26, C12Q 1/24

(54) **SIMPLE PORTABLE BACTERIA DETECTOR**

(30) Priority: 04.02.2004 JP 2004027972
(71) Applicant: SRL, INC., Tachikawa-shi, Tokyo 190-8567 (JP); Eiken Kizai Co., Ltd., Kita-ku Tokyo 114-0002 (JP)
(72) Inventor: MIYAMOTO, Toshihiko, Reagents SRL, Inc., Hachioji-shi, Tokyo 192-0032 (JP); ABE, Yoshihiko, Reagents SRL, Inc., Hachioji-shi, Tokyo 192-0032 (JP); IWAHORI, Tomoyuki, Reagents SRL, Inc., Hachioji-shi, Tokyo 192-0032 (JP); NAKAJIMA, Ayako, Eiken Kizai Togane Plant, Togane-shi, Chiba 283-0062 (JP); KITAGAKI, Tetsuo, Eiken Kizai Togane Plant, Togane-shi, Chiba 283-0062 (JP)
(74) Representative: Helbing, Jörg
(86) International application number: PCT/JP2005/001864
(87) International publication number: WO 2005/075624

(57) **Abstract**

A portable bacteria detector not only simply capable of inspecting the presence of food-poisoning bacteria and the like but also provided with a disinfecting or sterilizing means itself has been widely used. As its application fields spread, the detector has been required to be more inexpensive and be constituted in a form allowing easier waste disposal. In a portable bacteria detector provided with a culture medium and a disinfectant or germicide, constituent members for the detector are formed of plastic materials which are easy to incinerate. Furthermore, once a sample has been collected and a process for detecting the presence of bacteria has been started, the detector can be operated in a completely closed system. In this bacteria detector, an external force is applied from a direction perpendicular to the axis of the detector to break off a fragile part inside the detector, thereby leading to the performance of a liquid chamber opening operation.

## Description

### FIELD OF THE INVENTION

The present invention relates to bacteria detectors which comprise bacteria culture media and disinfecting or sterilizing means and can be safely and simply discarded after being used. The bacteria detectors allow unskilled persons or distributors who are not experts in this field to easily and safely inspect the presence of contamination with food-poisoning bacteria and the like in restaurants, cafeterias, catering establishments, refreshment stands, grocery stores, food and drink manufacturing or processing plants, home, and other places.

### BACKGROUND OF THE INVENTION

Recently, sanitary conditions have been improved. However, there are still too many patients who have suffered from serious diseases or have been led to death as a result of ingesting food or drink contaminated with pathogenic bacteria, in particular, food-poisoning bacteria. There is a demand for developing means that allow unskilled persons or distributors who are not experts in this field to easily detect the presence of bacteria which may cause problems in circumstances where food and drink are handled. Such means are desired not only at home but also in restaurants, cafeterias, catering establishments, refreshment stands, grocery stores, food and drink service industries, and food and drink manufacturing or processing plants from the viewpoint of product liability. In order to achieve this, there is provided a bacteria detector that can rapidly and safely detect and identify contamination with pathogenic bacteria, in particular, food-poisoning bacteria by a simple operation (Patent Document 1). The bacteria detector is safely and easily discardable after being used. Furthermore, the bacteria detector is also portable so that the device is suitable to being used personally or domestically.

The presence of food-poisoning bacteria can be voluntarily examined with such a portable bacteria detector. The examination can be simply and easily carried out without utilizing specialized agencies such as the healthcare center. Consequently, the number of bacteria detector users has been increasing. With an increase in the use, another bacteria detector having substantially the same configuration as that of the above-mentioned detector is also proposed (Patent Document 2). Under such a circumstance, it is required that used bacteria detectors can be simply and easily discarded and, in addition, that more inexpensive and more safely operable detectors can be provided.
[Patent Document 1] Japanese Unexamined Patent Application Publication No. H11-42080 (JP, 11-42080, A (1999))
[Patent Document 2] Japanese Unexamined Patent Application Publication No. 2002-281955 (JP, 2002-281955, A (2002))

### SUMMARY OF THE INVENTION

Recently, a waste reduction issue is a social problem. For waste reduction, consumer goods to be discarded must be properly segregated. In conventional bacteria detectors, glass ampules are used as receptacles for storing a liquid medium or a disinfectant. Therefore, the used bacteria detectors are waste inevitably containing plastic materials and glass materials. Consequently, as the consumption of the detectors increases, a waste treatment issue may occur. Additionally, a further improvement of the detectors is demanded from the viewpoint of reducing the burden on the environment.

In the simple portable bacteria detector, not only a liquid medium but also a disinfectant solution is stored in receptacles disposed inside the detector. The receptacles are broken off to draw these solutions therefrom. However, the receptacles should not be directly touched while they are broken off or torn to draw the solutions out. It is attempted to use a receptacle composed of a basal receptacle member and a sheet cap. A solution is drawn by punching the cap with a puncher. However, in such a receptacle, since only one opening is formed, the solution is insufficiently drawn out of the receptacle. In addition, the inside area of each used detector may be incompletely disinfected. Furthermore, even when the receptacles for the liquid medium and the disinfectant solution are composed of the basal receptacle member and the sheet cap, the sheet cap is made of materials containing metal foil, such as laminated materials. Therefore, the waste disposal of the used bacteria detector is still troublesome.

In the bacteria detector, a sample to be tested is subjected to cultivation and then the presence of pathogenic bacteria is checked and detected. Therefore, once the culture has been started, it is required that the detector is incubated, disinfected or sterilized and disposed in a substantially closed system. Accordingly, it is preferable that the detector have a structure which must comply with requirements with respect to the safety and the easiness of the operation.

The present inventors have conducted extensive studies on a simple portable bacteria detector having a structure that is easy to use and convenient to discard after being used. As a result, the inventors have found a structure that can store a liquid medium and a disinfectant in a completely closed system isolated from the external environment. In the bacteria detector of the present invention, all constituent members for the detector are formed of plastic materials. Thus, the present inventors have succeeded in developing the simple portable bacteria detector that can be safely used. Once a sample has been collected, not only the supply of a liquid medium and the disinfection of the used medium, but also the waste disposal of the used detector can be performed in a substantially closed system. Furthermore, the solution in the bacteria detector can be readily drawn to a certain container even if an external force is indirectly applied to the detector.

The present invention provides:
[1] A portable bacteria detector constituted of a hollow container having an opening and
   a cap assembly comprising an engaging portion that can engage with the opening of the hollow container to form a closed system isolated from the external environment, wherein
   said hollow container provides a space for culturing bacteria, and
   said cap assembly is capable of not only detachably engaging with the opening of the hollow container but also hermetically sealing the hollow container,
   said cap assembly being equipped with a sample-collecting member (sample-collecting portion or sample collector) in a fashion capable of being inserted into and drawn from the hollow container,
   said bacteria detector comprising
   (a) a mechanism or structure serving as a storage space for storing a culture medium used for cultivation of bacteria to be detected, in such a manner that the culture medium is not in contact with the sample collector until the incubation of the sample is started while the sample collector can be brought into contact with the culture medium for cultivation of bacteria to be detected when an external force is applied to a portion for storing the culture medium upon initiation of bacterial cultivation, and
   (b) a mechanism or structure serving as a storage space for storing a disinfectant or germicide in such a manner that the disinfectant or germicide is not in contact with the culture medium until the disinfection or sterilization of the used culture medium is performed while said disinfectant or germicide can be brought into contact with the used culture medium when an external force is applied to a portion for storing the disinfectant or germicide upon initiation of disinfective or sterile performance;
      (1) the cap assembly being formed of a synthetic resin so as to be hollow in shape and having a structure allowing the cap assembly, other than the portion engaging with the opening of the hollow container, to be closed or hermetically sealed against the external environment wherein a hollow space of the cap assembly is capable of communicating with a sample collector-accepting space in the hollow container;
      (2) the hollow space of the cap assembly comprising
         a means for forming at least two independent chambers for liquid, including a first chamber for liquid, formed by a first partition member and a first wall portion of the cap body, wherein said first chamber contains a first filled liquid and a second chamber for liquid, formed by a second partition member and a second wall portion of the cap body, wherein said second chamber contains a second filled liquid,
         a first opening-forming means for applying an external force to the first wall portion to form an opening (gap or hole) in the first liquid chamber,
         and
         a second opening-forming means for applying an external force to the second wall portion to form an opening (gap or hole) in the second liquid chamber; and
      (3) the first and second opening-forming means each comprising an opening-forming part provided at a portion of the partition member so that the opening-forming part will be opened when an external force is applied outside the wall of the cap body.
[2] The portable bacteria detector according to the above [1], wherein the opening-forming part of the partition member comprises a stick-like protrusion extending in the axial direction of the bacteria detector upwardly from a partition wall portion of the partition member and a thin-walled fragile part formed at a root of the protrusion.
[3] The portable bacteria detector according to the above [1] or [2], wherein the cap assembly has an approximately tubular hollow deformable body formed of a synthetic resin, comprising an opening-forming part which allows the formation of an opening (gap or hole) in said thin-walled fragile part by abutting the wall of the cap assembly body against the stick-like protrusion in response to an external force applied in a direction approximately perpendicular to the axis of the detector.
[4] The portable bacteria detector according to any of the above [1] to [3], wherein the first liquid is a culture medium and the second liquid is a disinfectant or a germicide.
[5] The portable bacteria detector according to any of the above [1] to [4], wherein the second partition member is provided with a concave at a hollow-container side thereof, and a tip of the stick-like protrusion provided for the first opening-forming means is movably fitted into the concave.
[6] The portable bacteria detector according to any of the above [1] to [5], wherein a cross section of the stick-like protrusion provided for the first opening-forming means, cut in a plane perpendicular to the axis of the bacteria detector, has a shape wherein the vertical length is unequal to the horizontal one.
[7] The portable bacteria detector according to any of the above [1] to [6], wherein a cross section of the first wall portion of the cap body, cut in a plane perpendicular to the axis of the bacteria detector, has a shape wherein the vertical length is unequal to the horizontal one, a cross section of the stick-like protrusion provided to the first opening-forming means, cut in a plane perpendicular to the axis of the bacteria detector, has a shape wherein the vertical length is unequal to the horizontal one, and the first wall portion of the cap body and the stick-like protrusion of the first opening-forming means are arranged so that the cross-sections thereof are similar in the direction of the shape.
[8] The portable bacteria detector according to any of the above [1] to [7], wherein a cross section of the stick-like protrusion provided to the second opening-forming means, cut in a plane perpendicular to the axis of the bacteria detector, has a shape wherein the vertical length is approximately equal to the horizontal one.
[9] The portable bacteria detector according to any of the above [1] to [8], wherein a cross section of the second wall portion of the cap body, cut in a plane perpendicular to the axis of the bacteria detector, has a shape wherein the vertical length is approximately equal to the horizontal one.
[10] The portable bacteria detector according to any of the above [1] to [9] , wherein the bacteria detector further comprises a movable protecting sheath disposed outside the second wall portion of the cap body.

### ADVANTAGEOUS PROFILES OF THE INVENTION

A portable bacteria detector of the present invention has a highly simplified configuration and can be formed by simplified members. Therefore, the bacteria detector can be manufactured from plastic (synthetic resin) materials only. Therefore, the bacteria detector can be discarded or incinerated without segregation of waste. Thus, costs (for manufacturing and waste disposal) can be remarkably reduced. Once a sample has been collected, the process for the detection can be performed in the completely closed system. Any handling of the detector members, for example, sliding a member toward a bacteria-culturing container, is not required. Since all joins of members other than that between a hollow container and a cap assembly are hermetically sealed, the detector is highly safe. In addition, the handling of the detector is very easy.

The above objects and other objects, features, advantages, and aspects of the present invention are readily apparent to those skilled in the art from the following disclosures. It should be understood, however, that the description of the specification including the following best modes of carrying out the invention, drawings, etc. is illustrating preferred embodiments of the present invention and given only for illustrative purposes. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

### BRIEF DESCRIPTION OF THE DRAWINGS

FIG. 1 shows longitudinal sectional views of a first embodiment of the bacteria detector according to the present invention.
FIG. 2 is an exploded perspective view depicting assembly of the constituent members for the bacteria detector according to the present invention.
FIG. 3 shows longitudinal sectional views of a second embodiment of the bacteria detector according to the present invention.
FIG. 4 shows cross-sectional views of the bacteria detector taken along the lines A-A' and B-B' in FIG. 3.
FIG. 5 shows longitudinal sectional views (bottom) of a first partition member 4 and a second partition member 5 constituting the bacteria detector of the present invention and plan views thereof (viewed from a tip 9 side of the bacteria detector).
FIG. 6 shows longitudinal sectional views of a bacteria detector having a configuration substantially similar to that of the first embodiment of the bacteria detector according to the present invention.
FIG. 7 shows longitudinal sectional views of a third embodiment of the bacterial detector according to the present invention.
FIG. 8 shows longitudinal sectional views depicting the third embodiment of the bacterial detector according to the present invention.

### Reference Numerals

- 21: hollow container
- 34: sample-collecting device
- 34a: sample-collecting portion
- 31: cap assembly
- 31a: cap assembly liquid-chamber-constituting wall member
- 31b: cap assembly container-engaging side constituent member
- 4: first partition member
- 5: second partition member

### BEST MODES OF CARRYING OUT THE INVENTION

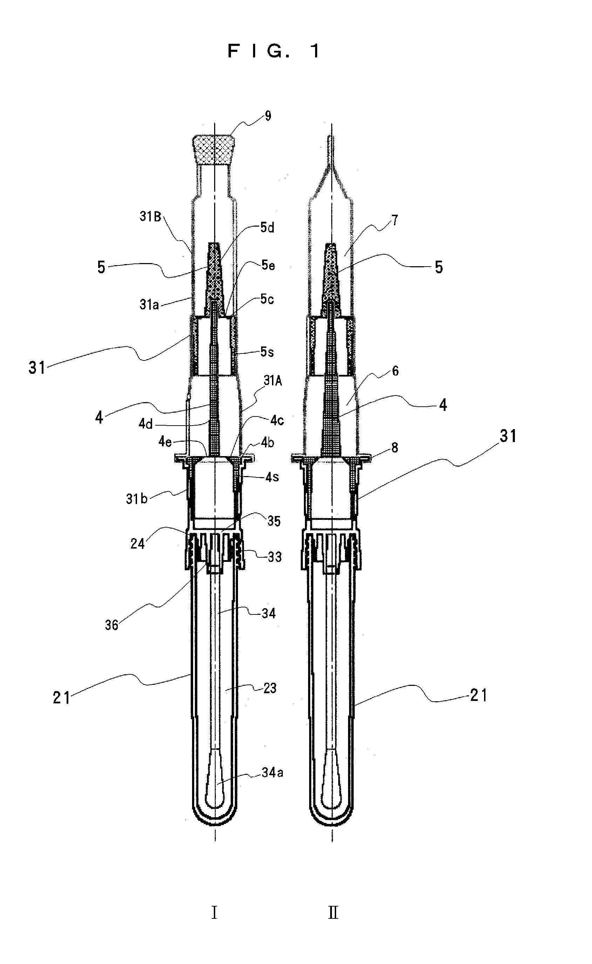

A first embodiment of bacteria detectors according to the present invention will now be described with reference to the drawings. FIG. 1 depicts longitudinal sectional views of the first embodiment of the portable bacteria detector according to the present invention. The sectional views are each taken along a plane containing the axis of the bacteria detector. The sectional views indicated by I and II in FIG. 1 are taken from directions which are orthogonal to each other with respect to the axis. In FIG. 1, the bacteria detector comprises a hollow container (preferably an elongated hollow container) 21 and a cap assembly 31. The cap assembly 31 includes a liquid-chamber-wall member 31a, a container-engaging member 31b, a first partition member 4, and a second partition member 5.

The hollow container 21 has an opening 22 that is engaged with the cap assembly 31. The hollow container 21 is hermetically sealed by the engagement between the opening 22 and the cap assembly 31 though the cap assembly 31 is detachable from the opening 22. A typical shape of the hollow container is a tube having a bottom, but the tube is not limited to a cylinder. The shape may be optionally determined from the viewpoints of manufacturing cost and simplicity of the manufacturing process. For example, the hollow container may have a body having a hexagonal cross-section, like a pencil, so that the container does not roll on a desk or may have a body partially having a hexagonal cross-section. Additionally, the body of the hollow container may be an approximately cylindrical tube having a flat portion or have an elliptical cross-section.

At a top of the hollow container 21, namely, at a side where the container is engaged with the cap assembly 31, an engaging portion is formed. The engaging portion is preferably formed in such a manner that the container is detachably engaged with the cap assembly while it can be readily joined to the cap assembly 31 to form a hermetic seal.

The configuration of the engaging portion may be optionally determined. The engaging portion is typically formed as a male screw. The container is hermetically sealed by the engagement of the male screw with a female screw formed at an engaging portion 33 provided to the cap assembly 31, though the container is detachable from the cap assembly. The screws may be opposite constitution, namely, a female screw may be formed at the engaging portion of the container, and a male screw may be formed at the engaging portion 33 of the cap assembly. Typically, the engaging portions are formed outside the hollow container 21 and inside the cap assembly 31. Reversely, the engaging portions may be formed inside the hollow container 21 and outside the cap assembly 31. The hermetic seal is achieved by the engagement between the hollow container and the cap assembly. Typically, the hermetic seal is attained by abutting a bottom face of the engaging portion 33 of the cap assembly 31 against a top end face 24 of the hollow container 21 and closely contacting them each other. The bottom face of the engaging portion 33 of the cap assembly 31 is a face on the hollow container side of the engaging portion at an end side opposed to the hollow-container. The shape of the top end face 24 of the hollow container viewed from the cap assembly side is a circle (O-shape) when the hollow container body is cylindrical in shape. Furthermore, the hermetic seal may be attained by closely and/or firmly contacting faces of the screw threads abutting on each other in cooperation with the above-mentioned firm connection.

In another configuration, the cap assembly may be provided with a groove on a container-engaging portion side so that an end of the hollow container fits in the groove. The engagement between the hollow container 21 and the cap assembly 31 is achieved by fitting the end of the container into the groove. Reversely, the hollow container may be provided with a groove on a cap-assembly-engaging portion side so that an end of the cap assembly fits in the groove. When the hollow container and the cap assembly are fittingly joined and/or closely contacted, the hollow container and the cap assembly may be each provided with one or more concaves and convexes on a surface of the engaging portion thereof. The joining (or close contact) is achieved by fitting the concaves and the convexes of the hollow container to the convexes and the concaves of the cap assembly, respectively. In such a configuration, the depth of the concave and the height of the convex formed on the surfaces may be small as long as the joining between the hollow container 21 and the cap assembly 31 can be achieved. The hollow container and the cap assembly are formed of a plastic material (synthetic resin). The engaging portions may injection molded when the hollow container or the cap assembly is formed out of plastic materials. The engaging portions may be formed of an elastic resin so that the joining and sealing performances are increased. The joining and sealing performances may be achieved by adjusting sizes of the engaging portions so that the joining and/or close contact between the hollow container 21 and the cap assembly 31 is attained by utilizing the elasticity of the plastic material.

When the hollow container 21 and the cap assembly 31 are joined together by fitting an end of one member in a concave at the engaging portion of the other member, the width of the concave of the other member may be made smaller than the thickness of the end of the one member. Thus, the joining and sealing performances in the fitting state may be achieved. In addition, a stopper mechanism may be provided to the bacteria detector so that the engagement between the hollow container 21 and the cap assembly 31 cannot be released after they have been engaged with each other after the collection of a sample. For example, when the hollow container 21 and the cap assembly 31 are packaged together in a bag, the hollow container 21 and the cap assembly 31 are engaged or can be readily released from each other. A sample-collecting device 34 is exposed and a sample-collecting portion (swab) 34a is rubbed against or brought into contact with a test target, such as cooking devices, to collect a sample. Then, the sample-collecting device 34 is inserted into a vacant room 23 (inside the container 21), and the cap assembly 31 is engaged with the hollow container 21. The stopper mechanism is actuated so that the cap assembly 31 is not detached from the hollow container 21 after the cap assembly 31 has been engaged with the hollow container 21. The hollow container 21 and the cap assembly 31 may be separately packaged in individual bags.

The hollow container 21 provides the vacant room 23 for incubating the sample. In the bacteria detector, after transferring the culture medium into the hollow container 21, the hollow container 21 is usually required to be kept standing (in other words, the axis of the hollow container is in the approximately vertical direction) during a period of time for inspecting the medium for grown bacteria, namely, during the incubation of the sample. A test-tube rack can be used for standing the hollow container 21 when the container body is cylindrical in shape like a test tube, but the method for standing the hollow container is not limited to. Any method is used as long as substantially equal effects can be achieved. In addition, the incubation may be performed in an incubator in which the hollow container 21 can be kept standing.

The cap assembly 31 comprises a liquid-chamber-wall member 31a, a container-engaging member 31b, a first partition member 4, and a second partition member 5. The first and second partition members are disposed inside the cap assembly. The container-engaging member 31b is detachably and sealingly mounted on the opening portion of the hollow container 21. The cap assembly 31 usually comprises a bottomless, hollow cap body composed of a body portion and a container-engaging portion extending from the body portion. The first partition member 4 and the second partition member 5 are disposed in the hollow space of the cap body. The cap assembly 31 provide a first chamber for liquid, and a second chamber for liquid, wherein said first chamber for liquid is enclosed with the wall of said cap body, that is, the first wall portion 31A thereof, the first partition member 4, and the second partition member 5, and said second chamber for liquid is enclosed with the second wall portion 31B of the cap body and the second partition member 5. The cap assembly has a hermetically sealed end at an opposite side to the container-engaging portion side. The end is sealed by fusing for the sake of manufacturing convenience.

At the upper side of the engaging portion 33 of the container-engaging member 31b, namely, at the liquid-chamber-wall member side of the engaging portion 33, a partition structure part 35 is provided in the neighborhood of the engaging portion 33. The partition structure part 35 provides a place capable of supporting a sample-collecting device 34 which is protrudingly positioned along the axis at approximately the center of the bacteria detector. Additionally, the partition structure part 35 is provided with a through-hole for allowing the migration of a liquid, such as a culture medium, from the cap assembly to the vacant space 23 of the hollow container. The partition structure part 35 is disposed in a fashion to axially partition the bottomless, hollow (ordinarily tubular) container-engaging member 31b at the middle portion. The partition structure part 35 may be formed of a plastic material according to techniques including injection molding, preferably together with the container-engaging member 31b. The plastic material used herein usually includes hard or poorly deformable plastic materials, such as polypropylene and ABS. The partition structure part 35 is provided with a supporting portion 36 for receiving the sample-collecting device 34. The sample-collecting device 34 is protrudingly mounted on the partition structure part 35 by insertedly fitting one end of the sample-collecting device 34 into the supporting portion 36. The sample-collecting device 34 comprises a sample-collecting part 34a at one end and serves as a swab extending in the axial direction of the hollow container 21. The sample-collecting device 34 is removably insertable into the vacant room 23.

Any structure may be optionally employed as the supporting portion 36 as long as the supporting portion 36 can attachedly support one end of the sample-collecting device 34. Commonly, the supporting portion 36 preferably comprises an annular ridge protruding toward the hollow container 21 and a deep groove provided at the center of the supporting portion 36 (i.e., a concave-shaped structure), but is not limited to. Typically, the sample-collecting device 34 and the supporting portion 36 are joined by insertedly fitting an end of the sample-collecting device 34 into the groove on the concave-shaped supporting portion 36.

The partition structure part 35 is provided with at least one through-hole (not shown). The through-hole is an aperture passing through the partition structure part from the liquid-chamber-wall member side to the hollow container 21 side of the container-engaging member 31b. The through-hole allows a liquid such as the culture medium supplied from the liquid chamber of the cap assembly to flow into the vacant space 23 of the hollow container. The structure of the through-hole may be optionally determined. The partition structure part may be provided with a plurality of the through-holes. In some cases, such a partition structure part provided with a plurality of the through-holes is preferable. Commonly, the through-hole is preferably formed in such a manner that the liquid can pass through the through-hole and smoothly flow from the cap assembly 31 to the hollow container 21 when the bacteria detector is placed in a state that the bottom of the hollow container is downward and the axis of the hollow container is in the approximately vertical direction. Considering from such a viewpoint, two or three through-holes are preferably provided. Typically, the partition structure part 35 is provided with the through-hole so that a culture medium 64 in the first liquid chamber can be dropwise added to the vacant space 23 through the through-hole when the first liquid chamber is opened.

The formation and configuration of the partition structure part may be not specifically limited as long as the sample-collecting device is attachable to the partition structure part and a liquid such as the culture medium can smoothly flow from the cap assembly to the vacant part of the hollow container. The partition structure part is preferably formed of a plastic material by techniques such as injection molding, together with the container-engaging member, from the viewpoint of manufacturing cost and others.

The container-engaging member 31b is preferably formed of a plastic material (synthetic resin), in particular, a colorless and transparent resin, a colored and transparent resin, or a translucent resin. The container-engaging member thus formed allows observing a state of a liquid such as the culture medium which is supplied through an opening (gap, hole or break) formed in the liquid chamber. In addition, usability of the bacteria detector can be improved. Specifically, the entire container-engaging member 31b is made of a transparent, highly stiff synthetic resin such as polystyrole. The transparent (or translucent) type bacteria detector is one of preferable embodiments of the present invention.

The liquid-chamber-wall member 31a is disposed, adjacent to the container-engaging member 31b, at an opposite side to the hollow-container side. The container-engaging member 31b and the liquid-chamber-wall member 31a are hermetically and cohesively joined with each other by fusing, which is a method generally applied to plastic materials. In other words, the hollow space inside the cap assembly is completely isolated from the external environment after an opening portion of the cap assembly is engaged with the opening 22 of the hollow container 21.

In the hollow space inside the cap assembly 31 wherein said hollow space is constituted of the liquid-chamber-constituting wall member 31a and the container-engaging side constituent member 31b, both the first partition member 4 and the second partition member 5 are disposed to form the first chamber for liquid and the second chamber for liquid. The first liquid chamber is arranged at the hollow-container side in the cap assembly 31. The first liquid chamber is constituted of the first partition member 4, the first wall portion 31A that constitutes the cap body, and the second partition member 5 disposed approximately at the middle portion of the cap assembly 31. The first partition member 4 is arranged so as to completely isolate one hollow space inside the cap assembly 31 from another space at the hollow container side. The second partition member 5 is arranged so as to completely isolate one hollow space inside the cap assembly 31 from another space at the hollow container side. In a preferable embodiment, as shown in FIG. 1, the first partition member 4 is arranged to be in contact with the inner wall of the container-engaging side constituent member 31b in a fashion capable of parting the hollow space inside the cap assembly 31 wherein said hollow space is constituted of the liquid-chamber-constituting wall member 31a and the container-engaging side constituent member 31b. Similarly, the second partition member 5 is arranged so that the hollow space formed in the cap assembly 31 at an opposite side to the hollow-container side has a sufficient capacity for storing a predetermined liquid. The second partition member 5 is arranged at a side opposite to and farther away from the hollow-container with respect to the first partition member 4 so as to part the hollow inside space enclosed with the liquid-chamber-constituting wall member 31a in the cap assembly. In this manner, the body wall of the cap assembly 31 is partitioned with the second partition member 5 into the second wall portion 31B and the first wall portion 31A extending from the container-engaging member 31b. The first liquid chamber is usually filled with a culture medium 64. The second liquid chamber is constituted of the aforementioned second partition member 5 and the second wall portion 31B of the cap body. The second liquid chamber is usually filled with a disinfectant or a germicide 74.

Typically, the portions 31b and 31A of the cap assembly 31 each have a tubular body form part. The diameters of such tubular body parts of the cap assembly portions 31b and 31A are set approximately equal. Between the portions 31b and 31A, a bonded part (area) 8 is present. In this embodiment, the parts 31b and 31A are joined to each other at the bonded part 8 where a slightly projecting part (flange-like construction) is formed.

The first partition member 4 is composed of a ring-like tubular part 4s, a ring-like protrusion 4b, a partition wall portion 4c, and a stick-like protrusion (elongated break-off bill) 4d. These parts are formed by injection molding. The ring-like tubular part 4s is airtightly, insertedly fitted to the entire inner circumference of the tubular part of the container-engaging member 31b and the inner circumference of the tubular body of the container-engaging member of the cap assembly 31. The ring-like protrusion 4b is formed on the outer circumference of the ring-like tubular part 4s. The partition wall 4c extends from the top end of the ring-like tubular part 4s with a sharp angle. The stick-like protrusion 4d extends in the axial direction from the central part of the partition wall 4c with a sharp angle. The root of the stick-like protrusion 4d is formed as a thin-walled fragile part 4e (including a thin rupturable or frangible wall). The second partition member 5 has a structure similar to that of the first partition member 4. The material for the tubular body of the liquid-chamber-constituting wall member is preferably selected so that an external force can be applied to the stick-like protrusion 4d or the like by pressing or bending the wall of the body. Typically, the tubular body is formed of a flexible synthetic resin such as a low-density polyethylene. The liquid-chamber-wall member may be formed of a colorless and transparent resin, a colored and transparent resin, or a translucent resin. The liquid-chamber-wall member thus formed allows observing a state of the liquid such as a culture medium. In addition, improved operability is attained for the inventive bacteria detectors. The material for each of the first and second partition members 4 and 5 is preferably selected so that the stick-like protrusions 4d and 5d are broken off at the thin-walled fragile sites 4e and 5e by applying a force in the direction perpendicular to each axis (a horizontal force in the drawing indicated by I in FIG. 1) and an opening (gap, hole or break) can be readily formed at the root of each of the stick-like protrusions. Typically, the first and second partition members 4 and 5 are each formed of a hard synthetic resin such as ABS. Each of these members may be also formed of a colorless and transparent resin, a colored and transparent resin, or a translucent resin. The partition members thus formed are preferable in some cases. In addition, improved operability is attained for such bacteria detectors. The transparent (or translucent) type bacteria detector is one of preferable embodiments of the present invention.

The ring-like protrusion 4b of the partition member 4 is sandwiched between the step-like portion (flange) of the container-engaging-side constituent member 31b and the hollow-container-side end (flange) of the tubular body of the liquid-chamber-constituting wall member 31a of the cap assembly. An opening of the container-engaging-side constituent member 31b (an opening at an opposite side to the hollow-container) is adjusted to the opening of the liquid-chamber-wall member. Under the condition that the ring-like protrusion 4b of the partition member 4 is sandwiched between the container-engaging member 31b and the liquid-chamber-constituting wall member 31a, the flanges of both members are joined together by fusing. Thus, the first partition member and the liquid-chamber-constituting wall member are fixed to the container-engaging side member of the cap assembly.

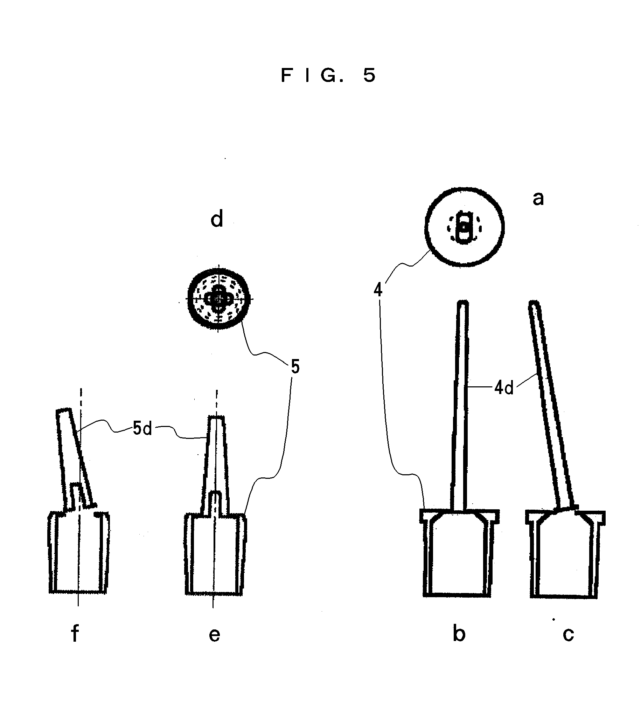

In the bacteria detector of the present invention, the hollow-container-side second partition member 5 has a concave part at the approximately central position (corresponding to the axis or near the axis area). A tip of the axially elongated stick-like protrusion 4d extends so that said tip is movably fitted in the concave part of the second partition member 5. With such a structure, the first wall 31A is pressed and deformed so that the stick-like protrusion 4d of the first partition member 4 can be applied with a force in a direction approximately perpendicular to the axis (the horizontal direction in the drawing indicated by I in FIG. 1). Consequently, the stick-like protrusion 4d is fractured and broken off at the thin-walled fragile part (usually thin rupturable or frangible wall) 4e at the root thereof (see FIG. 5c). Thus, a simple operation leads to the reliable opening of the first liquid chamber at the hollow-container side. In other words, since the tip of the stick-like protrusion 4d is movably fitted in the concave part provided to the second partition member 5, the horizontal force applied to the body of the stick-like protrusion 4d from a direction approximately perpendicular to the axis is concentrated on the thin-walled fragile part 4e. As a result, an opening (gap, hole or break) is formed, without fail, in the liquid chamber at the hollow-container side thereof by a slight force. Furthermore, the breakage of the stick-like protrusion 5d does not occur until the stick-like protrusion 4d has been broken off to open the first liquid chamber.

The liquid-chamber-wall member 31a has a hermetically sealed end 9 at an opposite side to the hollow-container side. The sealed end 9 may be formed, for example, as follows:
arranging the second partition member 5 in the inside hollow space of the liquid-chamber-wall member 31a so that the body wall of the liquid-chamber-wall member 31a can be partitioned into the first wall 31A and the second wall 31B;
filling a chamber formed by the second wall 31B and the second partition member 5 with a disinfectant or a germicide (usually, a liquid); and
then joining together the end of the liquid-chamber-wall member 31a at the opposite side to the hollow-container side by fusing, conventionally applied to plastic materials, to form a hermetic seal at the end site.

Thus, the hollow space of the cap assembly is completely insulated or isolated from the external environment.

The second partition member 5 is composed of a ring-like tubular body part 5s, a partition wall portion 5c, and a stick-like protrusion 5d, which are formed by injection molding. The ring-like tubular body part 5s is airtightly insertedly fitted into the entire inner circumference of the liquid-chamber-constituting wall member tubular part 31B and the inner circumference of the liquid-chamber-constituting wall member tubular body of the cap assembly 31. The partition wall portion 5c extends from the top end of the tubular body part 5s with a sharp angle. The stick-like protrusion 5d axially extends from the central site of the partition wall portion 5c with a sharp angle. The root of the stick-like protrusion 5d is formed as a thin-walled fragile part (usually, a thin rupturable or frangible wall) 5e. The material for the tubular body of the liquid-chamber-constituting wall member is preferably selected so that an external force can be applied to the stick-like protrusion 5d by pressing or bending the second wall 31B.

In the bacteria detector thus constituted, the sample-collecting portion 34a used for collecting a sample and the stick-like sample-collecting device 34 are inserted into the hollow container 21. The engaging portion 33 of the cap assembly 31 is sealingly fitted into the opening of the hollow container 21 (as a cork is put in a bottle). The first wall 31A of the cap body is then pressed and deformed so that the stick-like protrusion 4d of the first partition member 4 can be pressed in a direction approximately perpendicular to the axis. As a result, the protrusion 4d is fractured and broken off at the thin-walled fragile part 4e at the root thereof to form an opening (gap) in the first liquid chamber 5. The force in the lateral direction may be applied to the stick-like protrusion 4d by bending the flexible body of the cap assembly. Thus, the first liquid chamber 6 will be communicated with the space inside the hollow container 21 via the spaces and communicating passages in the tubular part 4s of the first partition member 4 and the tubular part of the container-engaging member 31b of the cap assembly 31. Then, a first liquid can be supplied to the sample on the sample-collecting portion 34a. The first wall 31A of the cap assembly 31 may be repeatedly pressed, if necessary. After the incubation is initiated, a predetermined detection process is performed. After the culture medium is added, a predetermined time passes, and detection for the presence of bacteria is performed, the culture medium is disinfected or sterilized. In order to perform the disinfection or sterilization process, the second wall 31B of the cap body is pressed and deformed so that the stick-like protrusion 5d of the second partition member 5 can be pressed in a direction approximately perpendicular to the axis. As a result, the protrusion 5d is fractured and broken off at the thin-walled fragile part 5e at the root thereof to form an opening (gap) in the second liquid chamber 7. The force in the lateral direction may be also applied to the stick-like protrusion 5d by bending the flexible body of the cap assembly. Thus, the second liquid chamber 7 comes to be communicated with the space inside the hollow container 21 via the spaces and communicating passages in the already opened first liquid chamber 6, the tubular part 4s, and the tubular body part 31b of the cap assembly 31. Then, a second liquid is added to the used culture medium in the container. The second wall 31B of the cap body may be repeatedly pressed, if necessary. In accordance with this embodiment, the first wall 31A of the cap body can be pressed and deformed for the addition of the first liquid, and independently the second wall 31B of the cap body can be pressed and deformed for the addition of the second liquid, thereby enabling the sequential supply of the first liquid, a culture medium, and the second liquid, a disinfectant or a germicide, to a sample inside a completely hermetically sealed container. The operation is simple not to require any skill. Therefore, once a sample has been collected, the processes for supplying the culture medium, disinfection or sterilization, and also disposal of the detector can be safely and simply performed without risk of exposure.

In accordance with this embodiment, constituent members for the bacteria detector can be formed of plastic materials. Therefore, the detector can be safely handled and readily discarded or burned up.

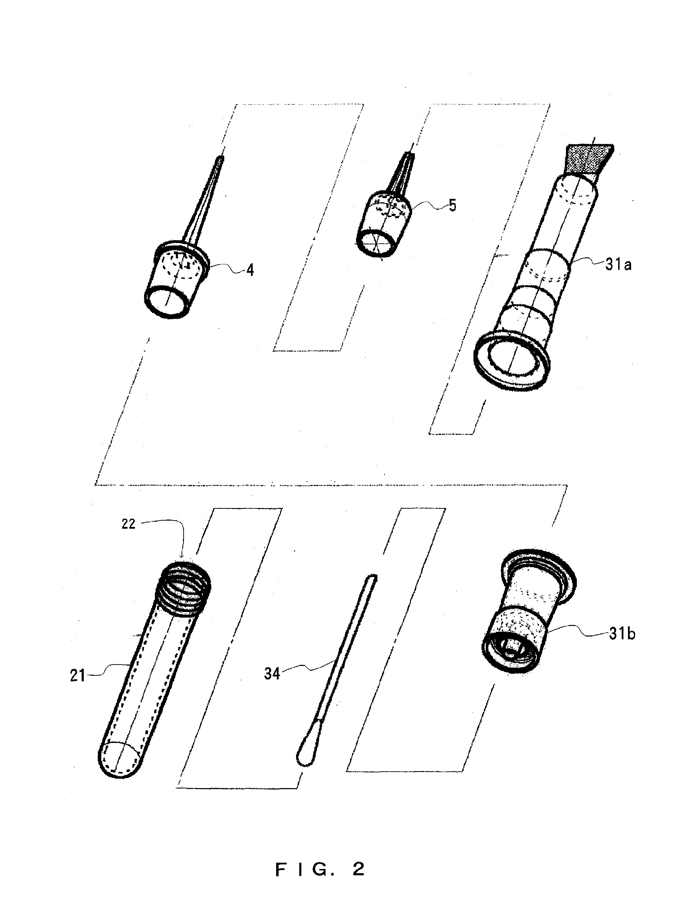

FIG. 2 is an illustrative drawing of assembling each constituent member for the bacteria detector according to the present invention. FIG. 2 shows a liquid-chamber-constituting wall member 31a of the cap assembly, a second partition member 5, a first partition member 4, a container-engaging side constituent member 31b, a sample-collecting device 34, and a hollow container 21. The bacteria detector of the present invention is formed of a small number of constituent members. Consequently, manufacturing cost can be significantly reduced or saved. Since the assembling of these members is simple, the manufacturing cost can be further reduced. In other words, as partially described above, the second partition member 5 is arranged inside the liquid-chamber-constituting wall member 31a which constitutes the cap assembly. The resulting space corresponding to the second liquid chamber is filled with a disinfectant or germicide liquid, and then the end is sealed (usually, sealed by fusing because the member is made of a plastic material). The second partition member 5 in combination with the wall 31A of the liquid-chamber-constituting wall member 31a forms a space corresponding to the first liquid chamber. The first liquid chamber is filled with a culture medium. Then, the first partition member 4 is arranged. The container-engaging side constituent member 31b is arranged so that the flange of the container-engaging member 31b can abut on the flange of the liquid-chamber-wall member 31a. The flanges are joined together (usually, joined by fusing because the members are each made of a plastic material). Then, the sample-collecting device 34 is attached to the container-engaging member 31b. Thus, the assembling of the bacteria detector is completed. However, it should be understood that this assembling process is only an exemplary embodiment for briefly illustrative purposes. Each step may be optionally carried out simultaneously or may be carried out in a modified way. Typically, the liquid-chamber-constituting wall member 31a, the second partition member 5, the first partition member 4, the container-engaging side constituent member 31b, the sample-collecting device 34, and the hollow container 21 are all formed of incineratable materials. For example, the liquid-chamber-wall member 31a and the container-engaging member 31b of the cap assembly may be formed of a flexible or readily deformable plastic material, such as polyethylene (PE). The first partition member 4 and the second partition member 5 may be formed of a hard or readily frangible or brittle plastic material, such as polystyrene (PS). A stick-like part of the sample-collecting device 34 may be formed of polypropylene and a sample-collecting portion 34a (swab) may be formed of a fiber mixture, for example, the mixture of cotton fibers and rayon fibers. The hollow container 21 is preferably formed of a transparent plastic material such as polystyrene (PS).

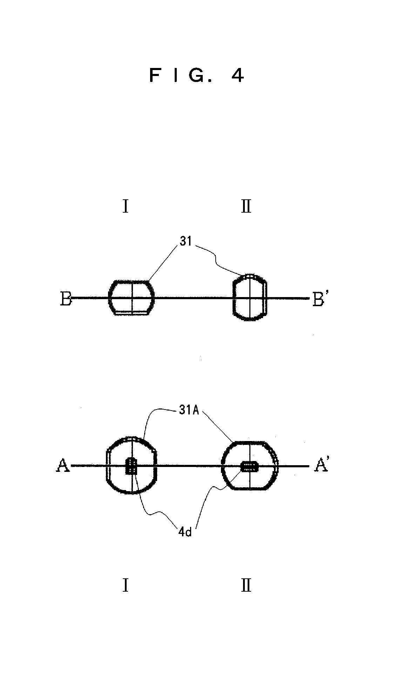

In a preferable embodiment, the protrusion 4d has a long stick-like shape (like a tall spire). The stick-like protrusion 4d is provided for a first opening means of this embodiment. As shown in a cross-sectional view taken along the line A-A' at the bottom of FIG. 4, the cross section of the stick-like protrusion, cut in a plane perpendicular to the axial direction of the bacteria detector, has a shape wherein the longitudinal length is different from the lateral one. With such a shape, for example, in the right case indicated by the line A-A' in FIG. 4, a force vertically applied to the cap body can certainly and reliably work on the fragile part at the root of the protrusion. Consequently, the protrusion is fractured and broken off at the fragile part. Thus, an opening can be securely and readily formed in the first liquid chamber at the hollow-container side. However, in this case (the right case indicated by the line A-A' in FIG. 4), it is relatively difficult to break the fragile part at the root of said protrusion and form an opening in the liquid chamber when the force is horizontally applied.

As shown in FIG. 5, the stick-like protrusion 4d is formed in such a shape that the cross section, cut in a plane perpendicular to the axis of the bacteria detector, has a longitudinal length different from lateral one (refer to each protrusion 4d as shown in the line A-A' right drawing of FIG. 4 and FIG. 5a). On the other hand, the stick-like protrusion 5d is formed in such a shape that the cross section, cut in a plane perpendicular to the axis of the bacteria detector, has a longitudinal length approximately equal to lateral one (refer to the protrusion 5d having a cross(+)-like shape in this case as shown in FIG. 5d). For the protrusion 4d, magnitudes of the external force necessary for breaking the fragile part can differ from force direction to force direction. In contrast, for the protrusion 5d, such a difference is not provided. Thus, the responses to the applied force become different between these protrudes 4d and 5d. As a result, it can be preferably prevented to improperly form an opening in one fragile part when an opening should be formed in another fragile part.

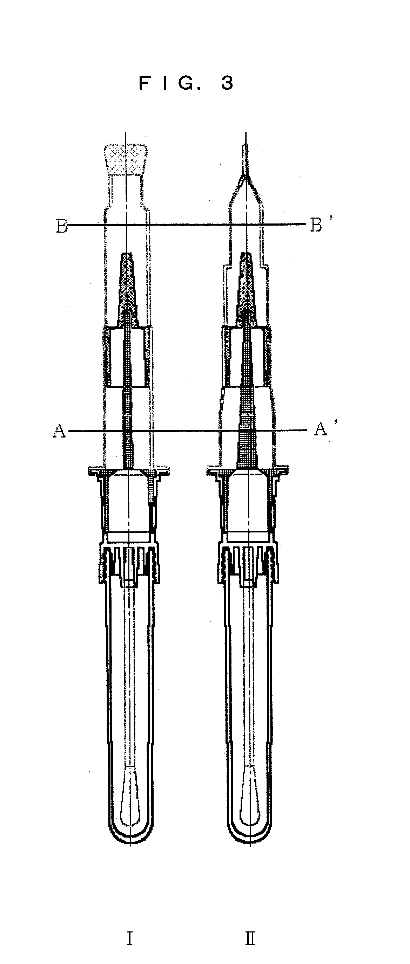

FIG. 3 shows longitudinal sectional views of a second embodiment of the portable bacteria detector according to the present invention. The cross-sectional views are each taken along a plane containing the axis of the bacteria detector. The views I and II in FIG. 3 are taken along planes which are orthogonal to each other with respect to the axis. FIG. 4 shows cross-sectional views taken along the lines A-A' and B-B' in FIG. 3, that is, cross-sectional views of a second wall portion 31B and a first wall portion 31A, which are constituents for a cap assembly 31, cut in planes perpendicular to the axial direction of the bacteria detector. In this embodiment, the first wall 31A and the second wall 31B are avoided to have shapes wherein the cross sectional shape of the first wall 31A is similar to that of the second wall 31B. Therefore, easiness in bending of the walls varies to each other with respect to the direction of the applied force. For example, in the left case indicated by the line A-A' in FIG. 4, the cap body is rarely bent with a vertically applied force, but is readily bent with a horizontally applied force. On the other hand, in the left case indicated by the line B-B' in FIG. 4, the cap body is rarely bent with a horizontally applied force, but is readily bent with a vertically applied force. In the bacteria detector shown in FIG. 3, it would be understood, by referring to the drawing indicated by II in FIG. 3, that the second wall 31B of the cap assembly has a body thickness, at the line B-B', smaller than that at the hollow-container side. By providing such a difference in the thickness, easiness in the bending of the body of the cap assembly will differ among positions where receive a horizontal force. Therefore, the protrusion 5d can be avoided to be accidentally broken off.

Hence, in the preferable portable bacteria detector, the first wall cross section of the cap body, cut in a plane perpendicular to the axial direction of the bacteria detector, may have a longitudinal length different from lateral one. In addition, a cross section of the stick-like protrusion for the first opening means, cut in a plane perpendicular to the axial direction of the bacteria detector, may have a longitudinal length different from lateral one. The cap body wall and the stick-like protrusion are arranged so that the cross sectional shape of the cap body wall is similar to that of the stick-like protrusion. Furthermore, in the portable bacteria detector, the second wall cross section of the cap body, cut in a plane perpendicular to the axial direction of the bacteria detector, may preferably have an approximately equal longitudinal length to lateral one, independently of the construction for the first wall of the cap body.

FIG. 5 shows enlarged views of the first partition member 4 (indicated by **a** to **c** in FIG. 5) and the second partition member 5 (indicated by **d** to **f** in FIG. 5) which are constituents for the bacteria detector of the present invention. These members are disposed in the hollow space of the cap body so that each chamber for liquid can be constituted. The drawings indicated by **a** and d in FIG. 5 are viewed from the top end 9 side of the bacteria detector. These drawings are shown mainly for helping to understand the cross sectional shapes of the (stick-like) protrusions 4d and 5d, cut in a plane perpendicular to the axis of the bacteria detector. The drawings indicated by **b** and **c** in FIG. 5 are longitudinal sections of the first partition member 4, viewed along a plane parallel to the axis of the bacteria detector. These drawings are shown for helping to understand the features when an opening is formed in the fragile part at the root of the (stick-like) protrusion 4d. The breakage of the protrusion 4d (the protrusion 4d is broken off at the root thereof) is generated by a force applied from the lateral direction. The drawings indicated by e and f in FIG. 5 are shown for helping to understand the second partition member 5 having features similar to the above.

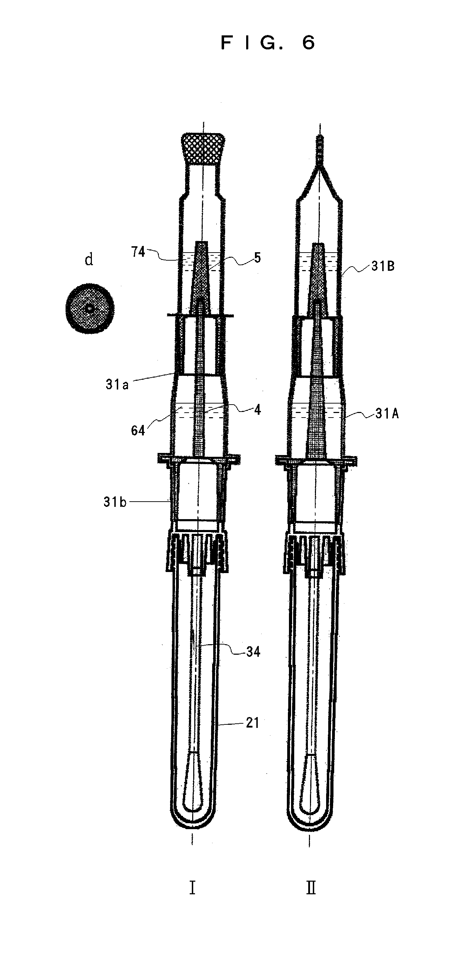

FIG. 6 illustrates longitudinal-sectional views of a bacteria detector having a configuration similar to that of the first embodiment of the portable bacteria detector according to the present invention. The sectional views indicated by I and II in FIG. 6 are taken along planes containing the axis of the bacteria detector and are viewed from directions orthogonal to each other with respect to the axis of the bacteria detector.

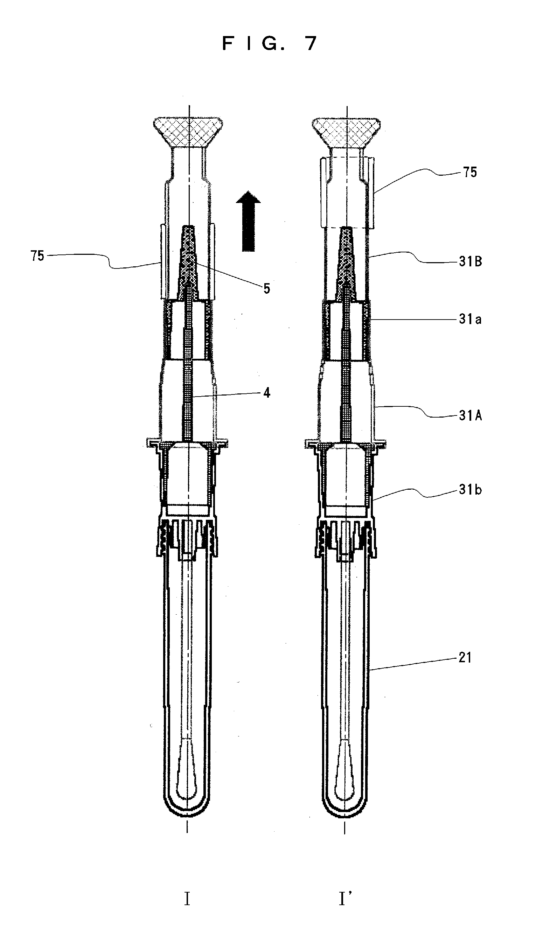

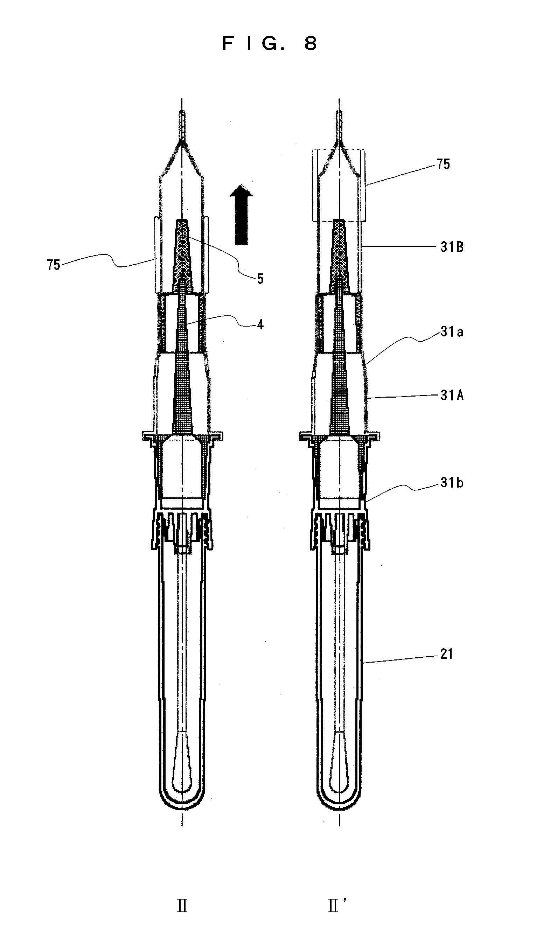

FIGS. 7 and 8 are longitudinal-sectional views of a third embodiment of the portable bacteria detector according to the present invention. The sectional views are taken along planes containing the axis of the bacteria detector. The drawings in FIG. 8 are viewed from a direction orthogonal to the direction from which the drawings in FIG. 7 are viewed, with respect to the axis of the bacteria detector. The drawing on the left in FIG. 7 shows a state that a protecting member (movable protecting sheath) 75 is mounted at a position for protecting the protrusion 5d. The drawing on the right in FIG. 7 shows a state that the protecting member is transferred to a position for enabling a force to act on the protrusion 5d. Correspondingly, the drawing on the left in FIG. 8 shows the state that the protecting member is placed at the position for protecting the protrusion 5d. The drawing on the right in FIG. 8 shows the state that the protecting member is transferred to the position for enabling a force to act on the protrusion 5d.

The size of the bacteria detector according to the present invention is not specifically limited. From the viewpoint of portability, the length is preferably about 5 to 30 cm, more preferably about 10 to 25 cm, and most preferably about 15 to 20 cm. The diameter is preferably about 7 to 30 mm, more preferably about 10 to 20 mm, and most preferably about 8 to 15 mm. The size of the bacteria detector, other than the above-mentioned sizes, may be optionally determined by those skilled in the art in consideration of the purpose, operability, usability, and quality of constituents for the detector.

The aforementioned embodiments are given for describing details of the present invention, but merely for illustrative purposes and for referential embodiments of the present invention. These exemplifications are intended to describe particularly specific embodiments of the present invention, but should not be construed as specifically defining or limiting the scope of the invention disclosed herein. It should be understood that various modifications and alterations can be made or executed within the spirit, scope and inventive concept disclosed herein.

All embodiments have been carried out or can be performed, unless otherwise disclosed herein specifically, by organizing and adapting standard technologies which are well-known and conventional to those skilled in the art.

In the present invention, bacteria detectors can be optionally equipped with a configuration suitably selected from various configurations disclosed in, for example, Japanese Unexamined Patent Application Publication No. H11-42080 (JP, 11-42080, A (1999)), in consideration of purposes and usability. Such bacteria detectors with adoptions of the selected configuration are also encompassed in the scope of the present invention.

### INDUSTRIAL APPLICABILITY

The bacteria detectors in accordance with the present invention allow performance of steps, after the collection of a sample, in a state that the entire device is completely hermetically sealed. The detectors are free from some apprehensions for leakage of contents from the joining parts or movable parts of each constituent member and remarkably much safer devices. Almost all constituent members for the bacteria detector can be formed of plastic materials. That is, materials which are troublesome in waste disposal or incineration, such as glass, are not contained in the detector. Therefore, the used detector can be conveniently discarded or incinerated without segregation of waste. Additionally, since the structures of the constituent members are simplified and the number of the members is reduced, manufacturing cost can be lowered and security can be also improved.

While the present invention has been described specifically in detail with reference to certain embodiments and drawings thereof, it would be apparent that it is possible to practice it in other forms. In light of the disclosure, it will be understood that various modifications and variations are within the spirit and scope of the appended claims.

## Claims

1. A portable bacteria detector constituted of a hollow container having an opening and
a cap assembly comprising an engaging portion that can engage with the opening of the hollow container to form a closed system isolated from the external environment,
wherein
said hollow container provides a space for culturing bacteria, and
said cap assembly is capable of not only detachably engaging with the opening of the hollow container but also hermetically sealing the hollow container,
said cap assembly being equipped with a sample-collecting member (sample collector) in a fashion capable of being inserted into and drawn from the hollow container,
said bacteria detector comprising
(a) a mechanism or structure serving as a storage space for storing a culture medium used for cultivation of bacteria to be detected, in such a manner that the culture medium is not in contact with the sample collector until the incubation of the sample is started while the sample collector can be brought into contact with the culture medium for cultivation of bacteria to be detected when an external force is applied to a portion for storing the culture medium upon initiation of bacterial cultivation, and
(b) a mechanism or structure serving as a storage space for storing a disinfectant or germicide in such a manner that the disinfectant or germicide is not in contact with the culture medium until the disinfection or sterilization of the used culture medium is performed while said disinfectant or germicide can be brought into contact with the used culture medium when an external force is applied to a portion for storing the disinfectant or germicide upon initiation of disinfective or sterile performance;
(1) the cap assembly being formed of a synthetic resin so as to be hollow in shape and having a structure allowing the cap assembly, other than the portion engaging with the opening of the hollow container, to be closed or hermetically sealed against the external environment wherein a hollow space of the cap assembly is capable of communicating with a sample collector-accepting space in the hollow container;
(2) the hollow space of the cap assembly comprising
a means for forming at least two independent chambers for liquid, including a first chamber for liquid, formed by a first partition member and a first wall portion of the cap body, wherein said first chamber contains a first filled liquid and a second chamber for liquid, formed by a second partition member and a second wall portion of the cap body, wherein said second chamber contains a second filled liquid,
a first opening-forming means for applying an external force to the first wall portion to form an opening in the first liquid chamber,
and
a second opening-forming means for applying an external force to the second wall portion to form an opening in the second liquid chamber; and
(3) the first and second opening-forming means each comprising an opening-forming part provided at a portion of the partition member so that the opening-forming part will be opened when an external force is applied outside the wall of the cap body.

2. The portable bacteria detector according to claim 1, wherein the opening-forming part of the partition member comprises a stick-like protrusion extending in the axial direction of the bacteria detector from a partition wall portion of the partition member and a thin-walled fragile part formed at a root of the protrusion.

3. The portable bacteria detector according to claim 1 or 2, wherein the cap assembly has an approximately tubular hollow deformable body formed of a synthetic resin, comprising an opening-forming part which allows the formation of an opening in said thin-walled fragile part by abutting the wall of the cap assembly body against the stick-like protrusion in response to an external force applied in a direction approximately perpendicular to the axis of the detector.

4. The portable bacteria detector according to any of claims 1 to 3, wherein the first liquid is a culture medium and the second liquid is a disinfectant or a germicide.

5. The portable bacteria detector according to any of claims 1 to 4, wherein the second partition member is provided with a concave at a hollow-container side thereof, and a tip of the stick-like protrusion provided for the first opening-forming means is movably fitted into the concave.

6. The portable bacteria detector according to any of claims 1 to 5, wherein a cross section of the stick-like protrusion provided for the first opening-forming means, cut in a plane perpendicular to the axis of the bacteria detector, has a shape wherein the vertical length is unequal to the horizontal one.

7. The portable bacteria detector according to any of claims 1 to 6, wherein a cross section of the first wall portion of the cap body, cut in a plane perpendicular to the axis of the bacteria detector, has a shape wherein the vertical length is unequal to the horizontal one, a cross section of the stick-like protrusion provided to the first opening-forming means, cut in a plane perpendicular to the axis of the bacteria detector, has a shape wherein the vertical length is unequal to the horizontal one, and the first wall portion of the cap body and the stick-like protrusion of the first opening-forming means are arranged so that the cross-sections thereof are similar in the direction of the shape.

8. The portable bacteria detector according to any of claims 1 to 7, wherein a cross section of the stick-like protrusion provided to the second opening-forming means, cut in a plane perpendicular to the axis of the bacteria detector, has a shape wherein the vertical length is approximately equal to the horizontal one.

9. The portable bacteria detector according to any of claims 1 to 8, wherein a cross section of the second wall portion of the cap body, cut in a plane perpendicular to the axis of the bacteria detector, has a shape wherein the vertical length is approximately equal to the horizontal one.

10. The portable bacteria detector according to any of claims 1 to 9, wherein the bacteria detector further comprises a movable protecting sheath disposed outside the second wall portion of the cap body.
